# EUROPEAN PATENT SPECIFICATION

(11) **EP 1 748 756 B1**
(45) Date of publication and mention of the grant of the patent: **29.04.2009**
(21) Application number: 05708751.2
(22) Date of filing: 10.03.2005
(51) Int. Cl.: A61K 9/00, A61K 9/107, A61K 9/48, A61K 9/16, A61K 9/20, A61K 9/14, A61K 9/70, A61K 31/565

(54) **COMPOSITIONS COMPRISING DROSPIRENONE MOLECULARLY DISPERSED**
ZUSAMMENSETZUNGEN AUS DROSPIRENON IN MOLEKULARER DISPERSION
DES COMPOSITIONS CONTENANT DROSPIRENONE A DISPERSION MOLECULAIRE

(30) Priority: 10.03.2004 EP 04075713; 10.03.2004 US 551355 P
(43) Date of publication of application: 07.02.2007
(60) Divisional application: 08011577.7
(73) Proprietor: Bayer Schering Pharma Aktiengesellschaft, 13353 Berlin (DE)
(72) Inventor: FUNKE, Adrian, D-14055 Berlin (DE); WAGNER, Torsten, 13127 Berlin (DE)
(74) Representative: Wagner, Kim
(86) International application number: PCT/IB2005/000665
(87) International publication number: WO 2005/087194

(56) References cited:
- EP-A- 1 260 225
- EP-A- 1 380 301
- WO-A-01/52857
- WO-A-20/04022065
- WO-A-20/04041289
- US-A- 5 569 652
- US-A- 5 656 622
- US-A- 5 789 442

## Description

The present invention relates to the field of pharmaceutical formulation science, in particular with respect to methods of improving solubility and bioavailability of lipophilic compounds, such as steroidal molecules, in particular with respect to drospirenone. The specific formulation technique of the present invention relates to a general principle of providing drospirenone in molecularly dispersed form.

### BACKGROUND

Un-formulated drospirenone is not well absorbed in the gastro-intestinal tract, partly because of its poor solubility in water and its low velocity of dissolution in water. Moreover, drospirenone has poor chemical stability in acidic environments including the condition provided in the gastric fluid of the stomach. In fact, almost 50% of the drospirenone is degraded into its therapeutically inactive isomer within 30 minutes when being contacted to a solution of hydrochloric acid with pH of about 1.0. Enteric coatings may be suggested in consequence thereof. It is considered that drospirenone in its nature of a steroidal molecule is to be absorbed in the upper part of the gastro-intestinal tract (GI tract), such as from the gastric mucosa and/or the mucosa in the duodenum, which requires that drospirenone should be completely dissolved already in the beginning of the GI tract.

It has been found that when providing drospirenone in micronised form, the in-vitro dissolution is high. For example at least 70% of drospirenone is dissolved at 30 minutes after start of dissolution testing. However, the micronisation technique requires special equipment, is costly and may be difficult to handle.

Alternative formulations to those including micronised forms of drospirenone may be needed.

Different formulation techniques are well-known for the preparation of pharmaceutical compositions having the active component in admixture with a liquid, a semi-solid or a solid excipient. However, such techniques merely aim at having very fine particles of the active substance uniformly dispersed with excipients and not to having the active compound dispersed at the molecular level with excipients. Furthermore, such formulation techniques do not imply a process for making a composition wherein the active compound in an initially critical step is dissolved in the carrier which then make up the final composition or at least a part of it.

WO0245693 describes dosage forms of active substances suitable for being applied whenever taste masking is desirable or damaging of coatings is to be expected. The dosage forms comprise the active substance as uniformly dispersed or dissolved in a matrix selected from the group consisting of paraffin, fatty alcohols, triglycerides, partial glycerides and fatty esters.

US 5,789,442 relates to the formulation of the active compoounds in admixture with conventional excipients, such as liquids, semi-liquids, solid organic or inorganic carriers, for parenteral or enteral administration.

US 5,569,652 relates to the formulation of active compounds by processing the active compound with vehicles and diluents.

US 5,656,622 relates to new derivatives of estradiol, which in combination with drospirenone may be provided as a capsule or tablet.

EP 1 260 225 relates to compositions comprising an estrogen.

WO 2004/041289 describes drospirenone in a pharmaceutical tablet preparation prepared by using conventional fluid-bed technology.

WO 2004/0222065 relates to compositions comprising a testosterone derivative and optionally a progestogen (drospirenone is mentioned), wherein the drug is in an aqueous or an oily suspension.

Still other techniques suggest using the active compound in micronised form in order to improve bloavallability by achieving fast dissolution of the active compound in water.

WO 01/52857 mentions compositions comprising drospirenone in that drospirenone is dissolved in a suitable solvent, e.g. methanol or ethyl actetate, before being sprayed onto the inert carrier.

Despite the fact that previous efforts already have resulted in some improvement of the bioavailability of per-orally administered drospirenone, these efforts have not eliminated the initial dissolution step of the drospirenone in the gastro-intestinal fluids before absorption of the drug can take place.

### SUMMARY OF INVENTION

Now provided is a pharmaceutical composition comprising at least one steroidal drug such as a progestin (e.g. drospirenone, progesterone, eplerenone, etonogestrel) and/or an estrogen (estradiol and esters thereof) in molecularly dispersed form. That is to say that the composition comprises a steroidal drug, preferably drospirenone, which is present in the composition in a non-particulate form. By being present in a molecularly dispersed form, it is intended that the drug is present in a dissolved state in the excipient. The molecularly dispersed drug will be released very fast as dissolution takes place instantly when the dosage unit has disintegrated. In a pharmaceutical composition wherein the drug is molecularly dispersed, the disintegration time is actually the rate-determining step for release of the drug, which means that the bioavailability can be significantly improved by the present invention.

Preferably the compositions are provided as either a solid or a semi-solid both forms are in turn preferably adapted for oral administration and preferably to be contacted with the gastric fluid and then absorbed from the gastro-intestinal tract. Such compositions are found to have high bioavailability, good chemical stability, and fast in-vitro dissolution release and can be produced under conditions, which do not require costly equipment or safety guards.

The present invention is directed not only to pharmaceutical compositions, but also to methods for preparation and to the use of the compositions.

The compositions of the invention are provided by a process comprising the steps of:
a) providing drospirenone and one or more carrier(s); and
b) dissolving the drospirenone completely in the one or more carrier(s); and
c) optionally, drying the mixture obtained in step b).

### DETAILED DESCRIPTION OF THE INVENTION

It has been found that pharmaceutical compositions comprising drospirenone in a molecularly dispersed form exhibit superior performance with respect to the manufacturing process, physical or chemical stability, in-vitro dissolution, in-vitro permeation of human colon cells and in vivo bioavailability.

In a first aspect, the invention provides a composition comprising drospirenone molecularly dispersed in a pharmaceutically acceptable carrier. In alternative terms, the invention encompasses a composition comprising drospirenone in dissolved form in a pharmaceutically acceptable carrier whether the composition being solid or semi-solid. A common feature of all compositions is that drospirenone is dissolved at the molecularly level in a pharmaceutically acceptable carrier and behaves like dissolved drospirenone without being a conventional solution of drospirenone. Typically, drospirenone cannot be detected by X-ray diffraction analysis or by other suitable methods such as light microscopy, electron microscopy or dissolution. As the drug is already present in a dissolved form, namely molecularly dispersed, the dissolution will take place instantly when disintegration of the dosage unit has taken place. Therefore a common feature of a dosage form is that when comparing disintegration time of the dosage form with the dissolution time, these will occur simultaneously within the uncertainties associated with the methods of analysis.

In alternative terms, the invention encompasses a composition comprising drospirenone on dissolved form in a pharmaceutically acceptable carrier whether the composition being solid or semi-solid. A common feature of all compositions is that drospirenone is dissolved at the molecularly level in a pharmaceutically acceptable carrier and behave like dissolved drospirenone without being a conventional solution of drospirenone.

Advantageously, the compositions of the invention exhibit rapid dissolution of drospirenone in vitro. Rapid dissolution is defined as the dissolution of at least 70% of drospirenone from the compositions at the time point of 30 minutes after start of in-vitro dissolution test, in particular at least 80% by weight of the drospirenone at the time point of 20 minutes after start of in-vitro dissolution test, even more preferably at least 85% by weight of the drospirenone at the time point of 15 minutes after start of in-vitro dissolution test. The test is carried out using as the dissolution medium 900 ml of water at 37°C and as the dissolution testing apparatus a USP XXVIII Paddle apparatus 2 operated at 50-100 rpm, such as 50 or 75 rpm.

As mentioned, high bioavailability may be achieved by using micronised drug. But this technology has some disadvantages because micronised drug tends to flocculate during production and the personnel handling it may easily be exposed to dust from it. These disadvantages can be avoided by the present production process, leading to a lower number of production steps, reduced dust formation during manufacturing, higher level of containment, higher level of environmental and occupational safety, reduced manufacturing costs, improved blend uniformity and improved dose uniformity.

Compositions of the invention are preferably in the form of a solid or a semi-solid.

Solid compositions according to the present invention may be referred to as solid solutions.

Semi-solid compositions according to the present invention are meant to define solutions with drospirenone on dissolved form, wherein the solvent has a viscosity and/or melting point determining whether the composition is a semi-solid composition.

The term "molecularly-dispersed" or "molecular dispersion" is used to describe any solid, semi-solid system in which a component A (such as drospirenone) is dispersed at the molecular level within another component B (such as a solvent or a polymer), so that component A can not be detected in crystalline form by X-ray diffraction analysis nor be detected as particulate form, be it in crystalline form or be it in amorphous form, by any microscopic technique. By the term "molecular dispersion" it should also be understood that drospirenone is dissolved in component B regardless of the nature of B. That is to say the term "molecularly dispersed" is interchangeable with the term "molecularly dissolved".

The term "solid dispersion" refers to a condition in which Drospirenone is in a substantially non-particulate form and is dispersed in a polymer matrix. This condition may also be referred to as "solid solution". Alternatively, Drospirenone is in crystalline form and is dispersed in a polymer matrix such that the crystals are being so fine that they cannot be detected by x-ray diffraction analysis. As used herein, the term "substantially non-particulate" refers to a condition in which more than 90% of Drospirenone is in non-particulate form.

Alternatively the absence of particulate matter, such as crystals and amorphous particles can be investigated by microscopy. As used herein, the term "substantially non-particulate" refers to a condition in which more than 90% of Drospirenone is in non-particulate form when analysing by means of microscopy.

The term "solid solution" is used to describe any solid system in which a component is dispersed at the molecular level within another.

In a particular form, a solid solution is characterised by having drospirenone (or another active ingredient) molecularly dispersed in a solvent that is absorbed onto a solid carrier capable of adsorbing the solvent while remaining solid. That is to say that drospirenone is completely dissolved in the solvent present on the surface of the carrier. Such solid solutions are formed by completely dissolving drospirenone in a solvent and then transforming the solvent into a solid by the addition of a solid carrier absorbing the solvent, while keeping drospirenone on dissolved form. A solid solution is said to contain drospirenone molecularly dispersed within the composition, more specifically within the solvent.

The term "Solution" is used to describe any semi-solid system in which a component is dispersed at the molecular level within another.

Thus, it is to be understood that compositions of the invention comprise drospirenone in non-particulate form, e.g. wherein drospirenone is not present in the composition in the form of micronised particles or particles in nano-size ranges. Hence, the term "molecularly dispersed" essentially excludes compositions wherein very fine particles of drospirenone, such as micronised particles or particles of nanosize, have been dry-mixed with pharmaceutically acceptable ingredients or carriers or wherein the final composition still comprise drospirenone in the form of particles.

The term "Supersaturated solution" is used to describe a solution containing a concentration of the drug that is higher than its saturation concentration when determined at room temperature. This is to say that despite the higher content of drospirenone no crystalline drug can be detected by powder x-ray diffraction analysis. Basically, supersaturated solutions are expected to be thermodynamically unstable leading to a saturated solution and recrystallized drug.

The term "Stabilised supersaturated solution" is used to describe a supersaturated solution wherein no recrystallized drug can be detected by x-ray diffraction analysis. For example, stabilisation can be achieved by adding a crystallisatlon-inhibiting agent.

The term "micro-emulsion" is used to describe a slightly opaque, opalescent, non-opaque or substantially non-opaque colloidal dispersion that is formed spontaneously or substantially spontaneously when its components are brought into contact with an aqueous medium. A micro-emulsion is thermodynamically stable and contains droplets or liquid "nano" size particles having a mean diameter of less than about 2 µm. Typically, a lipophilic drug incorporated in a micro-emulsion is present in dissolved form inside the mentioned droplets or liquid "nano" size particles.

The term "micro-emulsion pre-concentrate" refers to a composition which spontaneously forms a micro-emulsion in an aqueous medium, for example, in water, for example on dilution of 1:1 to 1:10, e.g. 1:10, or in the gastric juice after oral application.

In one embodiment, the pharmaceutically acceptable solvent is a semisolid at room temperature and/or has a melting point below 40°. Such solvents may be selected from the list h) to u) shown below.

Generally speaken, solvents for use in the semi-solid compositions of the invention and which are semi-solid at room temperature include but are not limited to: ethanol, isopropanol, glycerol, propylene glycol, transcutol ® (ethylendiglycolmonoethylether), polyols, citric acid esters, monoglycerides, diglycerides, vegetable oils, vegetable fats, partial synthetic triglycerides (e.g. medium chain triglycerides (MCT) such as miglyol®), synthetic triglycerides, mixtures of glycerol fatty acid esters such as Imwitor®, fatty alcohols, fatty alcohol ethers, fatty acids, fatty acid esters, waxes, paraffin, purified water and mixtures thereof. In addition, surfactants and co-solvents can be used.

Those skilled in the art will recognise that the melting point of several of the above-mentioned solvents depends on e.g. the length of hydrocarbon chains and the degree of substitution. Therefore, the skilled person will easily be able to select the proper solvent in order to produce a liquid or semi-solid composition.

Preferably the solvents are selected from the group comprising ethanol; propylene glycol; partial synthetic triglycerides; or vegetable oils.

Typically examples on polyols are Glycerol, propylene glycol, sorbitol, mannitol, inositol, pentaerythritol, maititol, lactitol.

Typically examples on citric acid esters are-tributylcitrat, triethylcitrat, acetyl-tributylcitrat, glyceryl stearate citrate.
Typically examples on monoglycerides are glycerol-monostearate, glycerol-monopalmitate, glycerol-monooleate, glycerol-monolinoleate.
Typically examples on diglycerides are-glycerol-dibehenate, glycerol-distearate.
Typically examples on vegetable oils are olive oil, peanut oil, castor oil.
Typically examples on fats are lard.
Typically examples on synthetic and partially synthetic triglycerides are neutral oil, softisan®, witepsol®, suppocire®.
Typically examples on glycerol fatty acid esters are Glycerolmonocaprylat, glyceryl laurate, caprylic/capric glycerides, glyceryl stearate
Typically examples on fatty alcohols are octanol, decanol, dodecanol, tetradecanol, hexadecanol, octadecanol, oleyl alcohol, linolyl alcohol, ricinol
Typically examples on fatty alcohol ethers, are oleyloleate, cetylpalmitate, ethyloleate,
Typically examples on fatty acids are capric acid, caprylic acid, lauric acid, palmitiylic acid, cetylic acid, stearylic acid, oleic acid, linolic acid
Typically examples on fatty acid esters are esters of lauric acid, caprylic acid, capric acid, stearic acid.
Typically examples on waxes are cera alba, beeswax.

Suitable surfactants include, but are not limited to:
a) Lecithine
b) block copolymers of ethylene oxide and propylene oxide such as Pkuronic® and Poloxamer® grades
c) glycerol esters, and polyoxyethylene glycerol esters, and mixtures thereof such as Gelucire®. Labrafil®, and Labrasol® grades
d) propylene glycol esters, such as Lauroglycol® and Capryol® grades
e) sucrose fatty acid esters such as Sucroesters®
f) sorbitan fatty acid esters, and polyoxyethylene sorbitan fatty acid esters, and mixtures thereof such as Span® and Tween® grades
g) polyoxyethylene fatty acid esters, polyoxyethylene fatty alcohol ethers, and polyoxyethylene mono-, di- and triglyceride esters, and mixtures thereof, such as Cremophor® grades.

In current interesting embodiments of the invention, the semi-solid compositions of the invention comprise as the solvent:
h) medium chain triglycerides
i) ricinus oil
j) Imwitor® 308 (Glycerolmonocaprylat)
k) Cremophor®EL
l) Cremophor® RH 40 (Polyoxyethylene-40-Glycerol-hydroxystearate)
m) polyethylene glycol 400
n) transcutol® P (ethylendiglycolmonoethylether)
o) Triethyl citrate
p) a mixture of 7% by weight glycerol and 93% by weight polyethylene glycol 400
q) a mixture of 50% by weight of Imwitor 308 and 50% by weight of polyethylene glycol 400
r) a mixture of 50% by weight of ricinus oil and 50% by weight of tributyl citrate
s) a mixture of 50% by weight of ricinus oil and 50% by weight of polyethylene glycol 400
t) a mixture of 75% by weight of Imwitor® 742, and 25% by weight of polyethylene glycol 400
u) a mixture of 75% by weight of Imwitor® 742, 15% by weight of polyethylene glycol 400 and 10% by weight of ethanol, or mixtures of any solvent h) to u).

Therefore, in some embodiments of the invention, the at least one pharmaceutically acceptable carrier is selected from the group consisting of medium chain triglycerides, ricinus oil, Glycerolmonocaprylat (Imwitor® 308), caprylaic/capric glycerides (Imwitor 742®), Polyoxyethylene-35-glycerol-triricinoleate (Cremophor EL®), Polyoxyethylene-40-Glycerol-hydroxystearate (Cremophor® RH 40), polyethylene glycol 400, ethylendiglycolmonoethylether (transcutol® P), triethyl citrate and mixtures thereof. In particular, a mixture of glycerol and polyethylene glycol 400, a mixture of Glycerolmonocaprylat (Imwitor® 308)and polyethylene glycol 400, a mixture of ricinus oil and tributyl citrate, a mixture of ricinus oil and polyethylene glycol 400, a mixture of caprylaic/capric glycerides (Imwitor® 742) and polyethylene glycol 400, a mixture of caprylaic/capric glycerides (Imwitor® 742) and polyethylene glycol 400 and ethanol.

Generally, the amount of drospirenone in 2 g of such semi-solid compositions is in the range of 1 mg to 30 mg, preferably 8 mg.

All these compositions can be encapsulated, e.g. in soft gelatine capsules.

In further embodiments of the invention, the composition is in the form of a micro-emulsion pre-concentrate. Such embodiments comprise in general the semi-solid solvent as mentioned above, but may further comprise at least one emulgator.

Suitable emulgators of the invention relate to polyoxyethylene-35-glycerol-triricinoleate (Cremophor® EL), polyoxyethylene-40-Glycerol-hydroxystearate (Cremophor^{®}RH40), polyoxyethylene-400-monoricinoleat, polyoxyethylene fatty acid glycerol esters (Gelucire®, Labrafil®, Labrasol®) and others. The emulgator may be mixed with suitable co-emulgators and/or co-solvent, such as ethylendiglycolmonoethylether (Transcutol^{®}P), Glycerolmonocaprylat (Imwitor^{®}308), and propylene glycol esters (Lauroglycol®, Capryol®).

In still other embodiments of the invention, the composition is in the form of a solid.

In one embodiment thereof, the composition comprises a pharmaceutically acceptable carrier that is a solid at room temperature and/or has a melting point above 40 °C, such as in the range of 40-80°C. As suitable solid carriers can be mentioned solid polyethylene glycols such as polyethylene glycol 6000, vegetable oils and fats, partial synthetic triglycerides, synthetic triglycerides, mixtures of glycerol fatty acid esters such as Imwitor®, mixtures of mono-, di and triglycerides, polyoxyethylene glycerol fatty acid esters, such as Gelucire®, fatty acids, fatty acid esters, waxes, paraffin, or mixtures thereof.

In some embodiments thereof, the molecularly dispersion comprises Drospirenone essentially uniformly dispersed in a matrix composed of a mixture comprising at least one fatty alcohol and at least one solid paraffin. In other embodiments, the matrix comprises at least one triglyceride and at least one solid paraffin or at least one partial glyceride and at least one solid paraffin or at least one fatty acid ester and at least one solid paraffin. In such preparations, the preparation preferably comprises microspheres.

In another form of a solid composition of the invention, the pharmaceutically acceptable carrier is a polymer. The polymer is preferably a hydrophilic polymer, which comprises free hydrophilic groups, such as polymers with functional groups in the side chains, such as free hydrophilic functional groups, such as carboxylic-, ester-, hydroxy-, amino-, amide, halogen- or sulfo- groups.

Typical examples on hydrophilic polymers which are water-soluble include, but are not limited to: polyvinylpyrrolidone (Povidone®, Kollidon®); polyvinyl acetate; polyvinyl alcohol; polyvinyl alcohol phthalate; polyethylene glycol (PEG); polyethylene oxide; gelatine; carborner; methacrylic acid copolymer; ammonio methacrylate copolymer; cellulose, carboxymethyl-cellulose; methyl cellulose; hydroxy ethyl cellulose; hydroxypropyl methylcellulose (HPMC), hydroxypropyl-cellulose (HPC); cellulose acetate phthalate; and hydroxypropyl methylcellulose phthalate; or copolymers or mixtures thereof.

The hydrophilic polymer may also be a water-insoluble polymer including, but are not limited to, crospovidone; sodium starch glycolate; and croscarmellose.

Preferably, the polymer is selected from the group comprising polyvinylpyrrolidone (Povidone®, Kollidon®), and polyethylene glycol (PEG), and polyvinylpyrollidone is particularly preferred. When a water-insoluble polymer is employed, crospovidone is preferred.

All of the foregoing polymers are well known in the art.

Polyvinylpyrrolidone represents polymers of I-vinyl-2-pyrrolidone. It is available commercially as Povidone or Kollidon having a weight average ranging from 2,000 to 1,500,000. Generally, the polyvinylpyrrolidone used has a weight average in the range of 7000 to 54,000, with 28,000 to 54,000 being preferred

Crospovidone represents water insoluble synthetic cross-linked homopolymers of N-vinyl-2-pyrrolidone. Generally, the crospovidone has a particle size of 20µm to 250µm, and preferably 50µm to 250µm (see, for example, Kollidon, polyvinylpyrrolidone for the pharmaceutical industry, by BASF).

Preferably, the ratio of drospirenone to polymer is 1:1 to 1:100, more preferably 1:2 to 1:20, and most preferably 1:5 to 1:10.

When the molecular dispersion of the present invention is prepared by dissolving Drospirenone and the polymer in an organic solvent or mixture of organic solvents, suitable organic solvents include, but are not limited to methylene chloride, methanol, ethanol, isopropanol, acetone, tetrahydrofuran, or mixtures thereof.

The solvent may be removed by conventional means: e.g., evaporating the solvent under a hood; use of a double drum dryer, or spray dryer or supercritical fluid extraction process.

It is to be understood that the present composition may also be applied to other drug molecules than drospirenone and to combinations of two or more types of drug molecules. Thus, the compositions of the invention may comprise any compound that is lipophilic and has a poor solubility in water at 25°C. In general, the compound has a solubility lower than 1 mg/ml in water at 25°C, such as lower than 0.5, 0.1, 0.05 or 0.01 mg/ml. Typically, the compound is a steroidal molecule and/or a hormone/antihormone in general. A large range of other active pharmaceutical ingredients may benefit from the present technology, such as albendazole, aminogluthethimide, aminosalicylic acids (3- 4- or 5-aminosalicylic acids) amiodarone, astemizole, azathioprine, beclamide, benorylate, benperidol, bezafibrate, biotin, bromocriptine, bromocriptine mesylate, bumetanide, busulphan, cabergoline, carbamazepine, cefixime, chenodeoxycholic acid, chlorambucil, chloroquine, chlorpropamide, chlorprothixene, chlorthalidone, cinnarizine, cinoxacin, clobazam, clofazimine, clofibrate, clonazepam, cyclopenthiazide, cyclosporin A, dapsone, demeclocycline, diazoxide, diflunisal, digitoxin, digoxin, disulfiram, domperidone, droperidol, enoxacin, epothilone, ethionamide, etretinate, felodipine, fenbufen, fexofenadine, flumazenil, folic acid, furosemide, glipizide, gliquidone, griseofulvin, haloperidol, hydrochlorothiazide, hydroflumethiazide, ibuprofen, iloprost, indomethacin, isocarboxazid, isosorbide dinitrate, isotretinoin, isradipine, itraconazole, ketazolam, ketoconazol, ketoprofen, lansoprazole, liothyronine sodium, lisuride, loperamide, loratadine, lorazepam, lovastatin, mebendazole, medazepam, mefenamic acid, menadione, mequitazine, methotrexate, misoprostol, morphine, niclosamide, nifedipine, nimodipine, nitrazepam, omeprazole, oxazepam, oxytetracycline, pantoprazole, perphenazine, phenylbutazone, pimozide, pindolol, probenecid, probucol, pyrantel embonate, pyrimethamine, retinol, riboflavin, simvastatin, stilboestrol, sulindac, sulphadiazine, sulphamethoxazole, sulphasalazine, sulpiride, tamoxifen, temazepam, thiabendazole, thioguanine, tocopherol, tolbutamide, tretinoin, triamteren, triazolam, trimethoprim, zopiclone.

As said, a compound of the invention may be a steroidal molecule or otherwise a hormone of which can be mentioned;
- androgens, such as testosterone and esters thereof (testosterone enanthate, testosterone undecanoate, testosterone cypionate, testosterone propionate)
- estrogens / antiestrogens, such as estradiol and esters thereof (estradiol valerate, estradiol enanthate, estradiol cypionate, estradiol undecylate), estriol, estrone, conjugated estrogens, equilin, ethinyl estradiol, fenestrel, mestranol, nylestriol, quinestrol, clomifene, estrogen receptor alpha agonists, estrogen receptor alpha antagonists, estrogen receptor beta agonists, estrogen receptor beta antagonists, estrogen receptor downregulators.
- Corticosteroids, such as cortisones and glucocorticoids, e.g. beclomethasone dipropionate, betamethasone, betamethasone valerate, budesonide, clobetasol propionate, clobetasone butyrate, cortisone acetate, dexamethasone, fludrocortisone acetate, prednisolone, prednisone.
- progestins/antiandrogens, such as cyproterone, etonogestrel, desogestrel, gestodene, levonorgestrel, norethisterones, norgestimate, norethindrone, norethindrone acetate, norethynodrel, norgestimate, norgestrel, medrogestone, medroxyprogesterone acetate, progesterone, progesterone receptor A specific ligands, progesterone receptor B specific ligands, mesoprogestins, antiprogestins, asoprisnil, asoprisnil ecamate
- Aldosterone antagonists, such as spironolactones, eplerenone, canrenoate, canrenone, dicirenone, mexrenoate, prorenoate, epostane, mespirenone, oxprenoate, spirorenone, spiroxasone, prorenone.
- Vitamin D hormones, such as alfacalcidol, calcifediol, calciferol, calcitriol.

It is to be understood that the compositions of the invention may comprise more than one active drug substance, e.g. a combination of two ore more drug substances. For example, a composition of the invention may comprise a therapeutic effective dose of drospirenone and a therapeutic effective dose of an estrogen.

### Form of pharmaceutical formulation

The composition comprising the molecular dispersion can, optionally, further comprise one or more additional active ingredient and/or excipients selected from the group comprising of: disintegrants, lubricants, glidants, artificial sweeteners, bulking agents, colorants and one or more flavorants.

The composition comprising the molecular dispersion can be produced in solid dosage forms. Solid dosage forms include tablets, film coated tablets, granules, pellets, pills, capsules and powders including for example any modified release form of said dosage forms such as dosage forms with delayed release coatings, sustained release coatings, enteric coatings, immediate release formulations, effervescent dosage forms and chewable forms. Capsules include e.g. soft gelatine capsules, hard gelatine capsules, hydroxypropyl methyl cellulose (HPMC) capsules, and carrageenan capsules.

In some embodiments, the compositions may be suitably formulated for buccal or sublingual administration. For example in the form of lozenges comprising Drospirenone in a molecular dispersion of a flavoured base or in the form of pastilles in an inert base of gelatine, glycerine, sucrose or acacia. Solid solutions of drospirenone in polymer films can also be placed directly onto the buccal mucosa without any further processing. Liquid compositions may be suitable for buccal or sublingual spraying application.

All dosage forms can be produced by methods well known in the art.

Typically, the amount of Drospirenone ranges from 1 to 50 wt% in the molecularly dispersed compositions, preferably from 5 to 50 wt%; and it ranges from 0.1 to 5.0 wt% in the pharmaceutical dosage form, such as tablets, granules, pellets or powders, preferably from 1.0 to 5.0 wt%. This is to say that typically the molecularly dispersed composition ranges from about 5 to 100 wt% in the pharmaceutical dosage form, preferably from 10 to 50 wt%.

### Excipients

As noted above, the compositions of the invention comprise a number of additional excipients than the required polymer, solvent, surfactant and crystallisation inhibitor.

Suitable disintegrants are selected from the group consisting of: croscarmellose sodium (a cross-linked polymer of carboxymethylcellulose sodium), crospovidone, starch NF; polacrilin sodium or potassium and sodium starch glycolate. Those skilled in the art will appreciate that it is desirable for compressible tablets to disintegrate within 30 minutes, more desirable within 10 minutes, most desirable within 5 minutes; therefore, the disintegrant used preferably results in the disintegration of the tablet within 30 minutes, more preferable within 10 minutes, most preferable within 5 minutes.

Suitable lubricants include talc, magnesium stearate, calcium stearate, stearic acid, hydrogenated vegetable oils and the like. Preferably, magnesium stearate is used.

Suitable glidants include pyrogenic silica, talc and the like.

Suitable bulking agents include xylitol, mannitol, compressible sugars, lactose, calcium phosphate and microcrystalline celluloses.

Suitable artificial sweeteners include saccharin, cyclamates and aspartame.

If desired, known flavorants and known FD & C colorants can be added to the composition.

### Process for preparing molecular dispersions of drospirenone

1. Compositions of the invention may be prepared by a process comprising the steps of:
   a) providing drospirenone and one or more carrier(s); and
   b) dissolving the drospirenone completely in the one or more carrier(s); and
   c) optionally, drying the mixture obtained in step b).

In the process described above, the step of dissolving of drospirenone in step b) is preferably performed by means selected from heating, ultrasonic treatment, vigorously mixing, stirring and/or melt extruding.

Drying of the mixture resulting from step b may include spray drying or other methods known in the art.

It should be understood that the process can be carried out using the pharmaceutically acceptable carriers mentioned above and the active compounds mentioned above.

A process and compositions for preparing micro-emulsion pre-concentrates is described in examples 1 to 6. Generally, a micro-emulsion pre-concentrate is prepared by a process comprising mixing a liquid or a semi-solid solvent or a mixture thereof and drospirenone under conditions securing complete dissolution of drospirenone in the liquid/semi-solid solvent, such as by use of an ultrasonic bath operated at 50°C. For examples on liquid or a semi-solid solvents see above, in particularly the solvents listed h) to u). The resulting pre-concentrate may contain from 1 mg drospirenone per 1500 mg concentrate to 1 mg per 100 mg concentrate, preferably 1 mg drospirenone per 1000 mg concentrate to 1 mg per 150 mg concentrate, such as 1 mg drospirenone per 750 mg or 250 mg concentrate.

Optionally at least one emulgator as mentioned above is also added to the liquid/semi-solid.

Upon adding water to the pre-concentrate an opalescent micro-emulsion is spontaneously formed that is ready to be administered. Typically, the amount of water added is from 2 ml to 100 ml per 1g of the concentrate, such as from 4 ml to 80 ml per 1g of the concentrate, such as from 5 ml to 70 ml per 1g of the concentrate.

The resulting micro-emulsion does not show any sedimentation or crystallisation for at least three days and centrifugation (6000 U, 10 min) does not cause any sedimentation or crystallisation.

Compositions according to examples 1 - 6 can be encapsulated in capsules. Basically, compositions according to examples 1 - 6 are preferably encapsulated in hard gelatine capsules that are sealed after filling.

Example 7 shows that the selection of solvents and surfactants in liquid and semi-solid compositions of the invention does not alter the velocity of dissolution and the permeability of drospirenone; drospirenone is quickly absorbed from all of the formulations investigated. However, surprisingly it was found that the formulation according to example 2 provides a lower degree of degradation of drospirenone.

Examples 8 to 12 and 23 describe the process for preparing solid solutions and preferred formulations:

Basically, there are two methods of preparing solid solutions. In one embodiment (example 11, 13 and 23) the active is mixed with a dry powdered polymer or a mixture of polymers and optionally an additive such as a surfactant like sucroester. This mixture is continuously extruded using a single screw extruder or a twin screw extruder or similar equipment at a rotation speed in the range of 10 to 100 rpm. Optionally, the temperature of the extruder can be controlled by heating and cooling, respectively, so that the process temperature is in a range of e.g. 40 - 150°C. During this process the whole mixture liquefies under the resulting pressure and temperature thus dissolving the active in the polymer. The resulting resolidified extrudate can be cut or broken into pieces, milled and sieved for further processing. The resulting in-vitro-dissolution data can be seen in example 24.

In another embodiment (examples 9 and 10) the solid solution is prepared via spray-drying from a solution containing the active and the polymer and optionally an additive such as a surfactant in dissolved form. Those skilled in the art know that spray-drying conditions have to be adjusted to the equipment used. The spray-dried product is further dried in a drying cabinet or via storage in an exsiccator using a drying agent such as phosphor pentoxide.

As mentioned earlier, the polymer is preferably a hydrophilic polymer, which comprises free hydrophilic groups, such as polymers with functional groups in the side chains, such as free hydrophilic functional groups, such as carboxylic-, ester-, hydroxy-, amino-, amide, halogen- or sulfo- groups. Typical examples on hydrophilic polymers which are water-soluble include, but are not limited to: polyvinylpyrrolidone (Povidone®, Kollidon®); polyvinyl acetate; polyvinyl alcohol; polyvinyl alcohol phthalate; polyethylene glycol (PEG); polyethylene oxide; gelatine; carbomer; methacrylic acid copolymer; ammonio methacrylate copolymer; cellulose, carboxymethyl-cellulose; methyl cellulose; hydroxy ethyl cellulose; hydroxypropyl methylcellulose (HPMC), hydroxypropyl-cellulose (HPC); cellulose acetate phthalate; and hydroxypropyl methylcellulose phthalate; or copolymers or mixtures thereof. The hydrophilic polymer may also be a water-insoluble polymer including, but is not limited to, crospovidone; sodium starch glycolate; and croscarmellose. Preferably, the polymer is selected from the group comprising polyvinylpyrrolidone (Povidone®, Kollidon®) and polyethylene glycol (PEG), and polyvinylpyrollidone is particularly preferred. When a water-insoluble polymer is employed, crospovidone is preferred.

Examples 13 - 16 describe a process for incorporating solid solutions into oral dosage forms such as tablets. A skilled person will easily imagine that similar formulations can also be filled into hard gelatine capsules. The preferred formulations basically include hydrophilic polymers as mentioned above in order to accelerate the partition of the drug to the intestinal fluid.

A process for preparing solid solutions in thin polymer films is described in example 17 Basically, a solution or suspension of active and polymers as described above and optional additives are drawn to a thin film on a film drawing machine using a knife in the range of 100 - 1000 µm. The resulting film is dried in a drying cabinet and cut into defined units. This kind of composition can be administered perorally (example 19) and to the buccal and sublingual mucosa as well (example 18). The latter provides the advantage that the dosage can easily be adapted to the individual need of every patient. The resulting in-vitro-dissolution data can be seen in example 25.

Another method of buccal and sublingual administration is described in examples 20 - 22 providing a solution of low to medium viscosity that is sprayed directly to the site of absorption.

### Examples

### Example 1

A micro-emulsion pre-concentrate is prepared by mixing 18.9 g Cremophor® RH 40 and 2.1 g Transcutol® P and 9.0 g medium chain triglycerides at 50°C. Then, 40 mg Drospirenone is added and mixed using an ultrasonic bath for 10 minutes at 50°C. 750 mg of the resulting micro-emulsion pre-concentrate contain 1 mg drospirenone.
When 100 mL of water is added, an opalescent micro-emulsion is formed spontaneously. The resulting micro-emulsion does not show any sedimentation or crystallisation for at least three days. Centrifugation (6000 U, 10 min) does not cause any sedimentation or crystallisation.
The same was observed upon addition of 2000 mL water.

### Example 2

A formulation prepared according to Example 1 using 120 mg Drospirenone instead of 40 mg has the same properties, but 250 mg of the resulting micro-emulsion pre-concentrate contain 1 mg drospirenone.

### Example 3

A micro-emulsion pre-concentrate is prepared by mixing 18.9 g Cremophor® EL and 2.1 g Transcutol® P and 9.0 g medium chain triglycerides at 50°C. Then, 40 mg Drospirenone is added and mixed using an ultrasonic bath for 15 minutes at 50°C. 750 mg of the resulting micro-emulsion pre-concentrate contain 1 mg drospirenone.
When 100 mL water is added, a slightly opaque micro-emulsion is formed spontaneously. The resulting micro-emulsion does not show any sedimentation or crystallisation for at least three days. Centrifugation (6000 U, 10 min) does not cause any sedimentation or crystallisation.
The same was observed upon addition of 2000 mL water.

### Example 4

A composition prepared according to example 3 using 120 mg Drospirenone instead of 40 mg has the same properties, but 250 mg of the resulting micro-emulsion pre-concentrate contain 1 mg drospirenone.

### Example 5

A micro-emulsion pre-concentrate is prepared by mixing 18.9 g Cremophor® EL and 2.1 g Transcutol® P and 9.0 g ricinus oil at 50°C. Then, 40 mg Drospirenone is added and mixed using an ultrasonic bath for 5 minutes at 50°C. 750 mg of the resulting micro-emulsion pre-concentrate contain 1 mg drospirenone.
When 100 mL water is added, an opalescent micro-emulsion is formed spontaneously. The resulting micro-emulsion does not show any sedimentation or crystallisation for at least three days. Centrifugation (6000 U, 10 min) does not cause any sedimentation or crystallisation.
The same was observed upon addition of 2000 mL water.

### Example 6

A composition prepared according to example 15 using 120 mg Drospirenone instead of 40 mg has same properties, but 250 mg of the resulting micro-emulsion pre-concentrate contain 1 mg drospirenone.

All these compositions as well as mixtures thereof can be encapsulated, e.g. in soft gelatine capsules.

### Example 7

Compositions containing drospirenone in molecularly dispersed form according to the examples 2, 4 and 6 were investigated in combined in-vitro dissolution - in-vitro permeation test. 250 mg of each composition was dispersed in 250 mL isotonic buffered salt solution. 1 mL of the resulting liquid was tested in a Caco-2 permeation study during 120 minutes. The principle of Caco-2 studies is well described in literature, e.g. Haltner E, Schmitz S, Gindorf C. In vitro Permeabilitätsuntersuchungen als Ersatz für Tier- und Humanstudien - welche Voraussetzungen müssen erfüllt sein? ALTEX 18(2001): 81-87; and Le Ferrec E, Chesne C, Artursson P, Brayden D, Fabre G, Gires P, Guillou F, Rousset M, Rubas W. In vitro models of the intestinal barrier: The report and recommendations of ECVAM workshop 46. ATLA Alternatives to Laboratory Animals. 29(2001): 649-668. Basically the permeation of compound across a human colon carcinoma cell monolayer is measured. In addition, gut metabolism and chemical stability with respect to the transformation of drospirenone into its inactive isomer is investigated. The In-vitro dissolution is determined using as the dissolution medium 900 ml of water at 37°C and as the dissolution testing apparatus a USP XXVIII Paddle apparatus 2 operated at 50 rpm.

The apparent permeability of drospirenone across human colon cells from all compositions investigated was in the range between 200 and 350 nm/s indicating a high permeability with no significant differences. However, differences were detected with respect to the extent of degradation by isomerisation of drospirenone. Degradation of drospirenone (i.e. transformation into inactive isomer) was measured initially ( after preparation of the compositions), - after the dissolution, before the start of the permeation study - and finally at the end of the permeation study, i.e. after 120 minutes contact with the colon cells:

| Composition | % DRSP degraded | | |
|---|---|---|---|
| | Initial | End | Difference |
| According to example 2 (RH40, Transcutol®, MCT) | 2.0 | 3.1 | + 1.1 |
| According to example 4 (EL, Transcutol®, MCT) | 3.8 | 4.7 | + 0.9 |
| According to example 6 (EL, Transcutol®, ricinus) | 4.8 | 5.4 | + 0.6 |

### Example 8

Semisolid compositions containing molecularly dispersed drospirenone are prepared by dissolving an amount of drospirenone in the range of 1 mg to 30 mg in a mixture of 1.05 g cera alba and 2 g medium chain triglycerides and 0.07 g lecithine by melting all components at about 100°C and stirring.
This composition can be encapsulated in soft gelatine capsules or hard gelatine capsules.

### Example 9

Solid solution of drospirenone prepared by spray drying:
1 g drospirenone, 8 g Kollidon® K30 and 1 g sucroester WE15 are dispersed in 300 mL purified water by stirring. The dispersion is heated to 60°C and continuously stirred until all excipients are dissolved. The resulting solution is spray-dried using a Mini Spray Dryer Büchl 190 (inlet temperature 120°C, outlet temperature 80°C, flow 4 g/min). The spray-dried product is stored for at least 24 h in an exsiccator using phosphor pentoxide as drying agent.

### Example 10

Solid solution of drospirenone prepared by spray drying:
1 g drospirenone, 8 g of a vinyl pyrollidone - vinyl acetat - copolymer (Kollidon® VA 64) and 1 g Gelucire® 44/14 are dispersed in 300 mL purified water by stirring. The dispersion is heated to 60°C and continuously stirred until all excipients are dissolved. The resulting solution is spray-dried using a Mini Spray Dryer Büchl 190 (inlet temperature 120°C, outlet temperature 80°C, flow 4 g/min). The spray-dried product is stored for at least 24 h in an exsiccator using phosphor pentoxide as drying agent.

### Example 11

Solid solution of drospirenone prepared by melt extrusion:
10 % drospirenone, 50 % polyvinyl pyrrolidone and 50 % saccharose monooleate are mixed. The mixture is continuously extruded using a single screw extruder at 60°C and 50 rpm.

### Example 12

Solid solution of drospirenone prepared by melt extrusion:
30 % drospirenone, 30 % polyethylene glycol 6000 and 40 % saccharose monooleate are mixed. The mixture is continuously extruded using a single screw extruder at 60°C and 50 rpm.

### Example 13

Compositions according to examples 9-11 can be processed to tablets. For that purpose, 300 g of the respective composition is blended with 490 g microcrystalline cellulose, than 10 g magnesium stearate is added and the mixture is again blended for 1 minute. The resulting mass is directly tabletted to give tablets of 80 mg tablet weight. Each single tablet contains 3 mg drospirenone.

### Example 14

Compositions according to examples 9-11 can be processed to tablets. For that purpose, 300 g of the respective composition is blended with 490 g lactose, then 10 g magnesium stearate is added and the mixture is again blended for 1 minute. The resulting mass is directly tabletted to give tablets of 80 mg tablet weight. Each single tablet contains 3 mg drospirenone.

### Example 15

Compositions according to examples 9-11 can be processed to tablets. For that purpose, 300 g of the respective composition is blended with 490 g microcrystalline cellulose, then 10 g pyrogenic silica is added and the mixture is again blended for 1 minute. The resulting mass is directly tabletted to give tablets of 80 mg tablet weight. Each single tablet contains 3 mg drospirenone.

### Example 16

100 g of the composition according to example 12 is blended with 395 g microcrystalline cellulose, then 5 g magnesium stearate is added and the mixture is again blended for 1 minute. The resulting mass is directly tabletted to give tablets of 50 mg tablet weight. Each single tablet contains 3 mg drospirenone.

### Example 17

1 g drospirenone and 1 g polyoxyethylene-polyoxypropylene copolymer are dissolved in 94 g ethanol by stirring. To the resulting solution is homogenously added a powder mixture of 17 g Hydroxypropylcellulose and 17 g Cellulose. The resulting suspension is drawn to a thin film on a suitable film drawing machine using a 500 µm knife. After drying a dry, whitish paper-like film is obtained having a thickness of about 180 µm. one unit of this film, i.e. a slice of about 3 cm² contains 1 mg drospirenone.

### Example 18

The composition according to example 17 can be cut into pieces of about 1 to about 15 cm² that are administered directly to the buccal mucosa, releasing 0.33 to 5 mg drospirenone to the buccal mucosa.

### Example 19

The composition according to example 17 can be cut into pieces that are filled into hard gelatine capsules for peroral application.

### Example 20

5 g Drospirenone, 1 g tocopherole acetate and 5 drops of peppermint oil are made up with ethanol to 1000 mL and dissolved by stirring. The resulting solution is filled into 10 mL glass bottles and covered by a pump spray nozzle. One application releases 0.1 mL solution containing 0.5 mg drospirenone.

### Example 21

5 g Drospirenone, 1 g ascorbyl palmitate, 50 g medium chain triglycerides, 300 g Cremophor® EL and 20 g Glycerolmonoester are made up with ethanol to 1000 mL and dissolved by stirring. The resulting solution is filled into 10 mL glass bottles and covered by a pump spray nozzle. One application releases 0.1 mL solution containing 0.5 mg drospirenone.

### Example 22

Solutions according to examples 20 and 21 are individually administered as a sublingual spray, e.g. 6x spraying release 3 mg drospirenone. The formulation according to example 22 provides a prolonged residence time on the buccal mucosa.

### Example 23

Solid solution of drospirenone prepared by melt extrusion:
10 % drospirenone, and 90 % polyethylene glycol 6000 are mixed. The mixture is continuously extruded using a single screw extruder at 60°C and 50 rpm.

### Example 24

30 mg of the formulation according to the example 23 was investigated in an in-vitro dissolution test using as the dissolution medium 900 ml of water at 37°C and as the dissolution testing apparatus a USP XXVIII Paddle apparatus 2 operated at 50 rpm. The Drosplrenone contained in the formulation was completely dissolved within 10 minutes.

Thus, it is understood that dissolution this fast takes place within the disintegration time of the dosage form. The dissolution step is therefore not the rate-determining step for release of the Drospirenone. It will be understood that in the present example the Drospirenone is therefore already present in a "dissolved form", such as molecularly dispersed as otherwise a dissolution this fast would not have been obtained.

### Example 25

The composition according to example 17 is cut into pieces of 9 cm² (containing 3 mg of Drospirenone) were subjected to an in-vitro dissolution test using as the dissolution medium 900 ml of water at 37°C and as the dissolution testing apparatus a USP XXVIII Paddle apparatus 2 operated at 50 rpm. The following results were found: Drospirenone released after 10 min: 95.0%, after 20 min: 97.1%, and after 30 min: 97.2%.

## Claims

1. A solid or semi-solid composition adapted for oral administration comprising Drospirenone molecularly dispersed in at least one pharmaceutically acceptable carrier.

2. The composition according to claim 1 in the form of a solid, wherein at least one of the pharmaceutically acceptable carriers is a solid at room temperature and/or has a melting point in the range of 40-80 °C and is selected from the group consisting of polyethylene glycol 6000 vegetable oils, vegetable fats, partial synthetic triglycerides, synthetic triglycerides, mixtures of glycerol fatty acid esters, mixtures of mono-, di and triglycerides, polyoxyethylene glycerol fatty acid esters, fatty acids, fatty acid esters, waxes, paraffin, and mixtures thereof.

3. The composition according to claim 1, wherein the pharmaceutical acceptable carrier is a polymer.

4. The composition according to claim 3, wherein said polymer is a hydrophilic polymer.

5. The composition according to claim 4, wherein the hydrophilic polymer is selected from the group consisting of polyvinylpyrrolidone; polyvinyl acetate; polyvinyl alcohol; polyvinyl alcohol phthalate; polyethylene glycol; polyethylene oxide; gelatine; carbomer; methacrylic acid copolymer; ammonio methacrylate copolymer; cellulose, carboxymethylcellulose; methyl cellulose; hydroxy ethyl cellulose; hydroxypropyl methylcellulose, hydroxypropyl-cellulose; cellulose acetate phthalate; and hydroxypropyl methylcellulose phthalate; crospovidone; sodium starch glycolate; croscarmellose; and copolymers thereof, and mixtures thereof.

6. The composition according to any of claims 1-5, wherein the composition further comprises an estrogen.

7. The composition according to any of the preceding claims, wherein said composition is in a solid dosage form.

8. A process for preparing a composition as defined in claim 1 comprising the steps of
a) providing Drospirenone and one or more carrier(s); and
b) dissolving the Drospirenone completely in the one or more carrier(s); and
c) drying the mixture obtained in step b).

9. The process according to claim 8, wherein the step of dissolving Drospirenone is performed by means selected from the group consisting of heating, ultrasonic treatment, vigorously mixing, stirring and melt extruding.

10. The process according to claim any of claims 8 or 9, wherein at least one of the pharmaceutically acceptable carriers is a solid at room temp and/or has a melting point in the range of 40-80 °C and is selected from the group consisting of polyethylene glycol 6000 vegetable oils, vegetable fats, partialsynthetic triglycerides, synthetic triglycerides, mixtures of glycerol fatty acid esters, mixtures of mono-, dl and triglycerides, polyoxyethylene glycerol fatty acid esters, fatty acids, fatty acid esters, waxes, paraffin, and mixtures thereof.

11. The process according to any of claims 8 or 9, wherein the pharmaceutically acceptable carrier is a polymer.

12. The process according to claim 11, wherein said polymer is a hydrophilic polymer.

13. The process according to claim 12, wherein the hydrophilic polymer is selected from the group consisting of polyvinylpyrrolidone; polyvinyl acetate; polyvinyl alcohol; polyvinyl alcohol phthalate; polyethylene glycol; polyethylene oxide; gelatine; carbomer; methacrylic acid copolymer; ammonio methacrylate copolymer; cellulose, carboxymethylcellulose; methyl cellulose; hydroxy ethyl cellulose; hydroxypropyl methylcellulose, hydroxypropyl-cellulose; cellulose acetate phthalate; and hydroxypropyl methylcellulose phthalate; crospovidone; sodium starch glycolate; croscarmellose; and copolymers thereof, and mixtures thereof.

## Patentansprüche

1. Feste oder halbfeste Zusammensetzung, die für die orale Verabreichung ausgelegt ist und Drospirenon molekular dispergiert in wenigstens einem pharmazeutisch unbedenklichen Träger enthält.

2. Zusammensetzung nach Anspruch 1 in Form eines Feststoffes, wobei wenigstens einer der pharmazeutisch unbedenklichen Träger bei Raumtemperatur fest ist und/oder einen Schmelzpunkt im Bereich von 40-80°C aufweist und aus der aus Polyethylenglykol 6000 Pflanzenölen, Pflanzenfetten, halbsynthetischen Triglyceriden, synthetischen Triglyceriden, Mischungen von Glycerinfettsäureestern, Mischungen von Mono-, Di- und Triglyceriden, Polyoxyethylenglycerinfettsäureestern, Fettsäuren, Fettsäureestern, Wachsen, Paraffin und Mischungen davon bestehenden Gruppe ausgewählt ist.

3. Zusammensetzung nach Anspruch 1, wobei es sich bei dem pharmazeutisch unbedenklichen Träger um ein Polymer handelt.

4. Zusammensetzung nach Anspruch 3, wobei es sich bei dem Polymer um ein hydrophiles Polymer handelt.

5. Zusammensetzung nach Anspruch 4, wobei das hydrophile Polymer aus der aus Polyvinylpyrrolidon, Polyvinylacetat, Polyvinylalkohol, Polyvinylalkoholphthalat, Polyethylenglykol, Polyethylenoxid, Gelatine, Carbomer, Methacrylsäurecopolymer, Ammoniummethacrylatcopolymer, Cellulose, Carboxymethylcellulose, Methylcellulose, Hydroxyethylcellulose, Hydroxypropylmethylcellulose, Hydroxypropylcellulose, Celluloseacetatphthalat und Hydroxypropylmethylcellulosephthalat, Crospovidon, Natriumstärkeglykolat, Croscarmellose und Copolymeren davon, und Mischungen davon bestehenden Gruppe ausgewählt ist.

6. Zusammensetzung nach einem der Ansprüche 1-5, wobei die Zusammensetzung weiterhin ein Östrogen umfaßt.

7. Zusammensetzung nach einem der vorhergehenden Ansprüche, wobei die Zusammensetzung in einer festen Dosierungsform vorliegt.

8. Verfahren zur Herstellung einer wie in Anspruch 1 definierten Zusammensetzung, bei dem man
a) Drospirenon und einen oder mehrere Träger bereitstellt, und
b) das Drospirenon vollständig in dem einen Träger bzw. den mehreren Trägern auflöst, und
c) die in Schritt b) erhaltene Mischung trocknet.

9. Verfahren nach Anspruch 8, wobei der Schritt des Auflösens von Drospirenon durch Erhitzen, Ultraschallbehandlung, kräftiges Mischen, Rühren oder Schmelzextrusion erfolgt.

10. Verfahren nach Anspruch 8 oder 9, wobei wenigstens einer der pharmazeutisch unbedenklichen Träger bei Raumtemperatur fest ist und/oder einen Schmelzpunkt im Bereich von 40-80°C aufweist und aus der aus Polyethylenglykol 6000 Pflanzenölen, Pflanzenfetten, halbsynthetischen Triglyceriden, synthetischen Triglyceriden, Mischungen von Glycerinfettsäureestern, Mischungen von Mono-, Di- und Triglyceriden, Polyoxyethylenglycerinfettsäureestern, Fettsäuren, Fettsäureestern, Wachsen, Paraffin und Mischungen davon bestehenden Gruppe ausgewählt ist.

11. Verfahren nach Anspruch 8 oder 9, wobei es sich bei dem pharmazeutisch unbedenklichen Träger um ein Polymer handelt.

12. Verfahren nach Anspruch 11, wobei es sich bei dem Polymer um ein hydrophiles Polymer handelt.

13. Verfahren nach Anspruch 12, wobei das hydrophile Polymer aus der aus Polyvinylpyrrolidon, Polyvinylacetat, Polyvinylalkohol, Polyvinylalkoholphthalat, Polyethylenglykol, Polyethylenoxid, Gelatine, Carbomer, Methacrylsäurecopolymer, Ammoniummethacrylatcopolymer, Cellulose, Carboxymethylcellulose, Methylcellulose, Hydroxyethylcellulose, Hydroxypropylmethylcellulose, Hydroxypropylcellulose, Celluloseacetatphthalat und Hydroxypropylmethylcellulosephthalat, Crospovidon, Natriumstärkeglykolat, Croscarmellose und Copolymeren davon, und Mischungen davon bestehenden Gruppe ausgewählt ist.

## Revendications

1. Composition solide ou semi-solide adaptée pour l'administration par voie orale, comprenant de la Drospirénone à dispersion moléculaire dans au moins un support pharmaceutiquement acceptable.

2. Composition selon la revendication 1 sous la forme d'un solide, dans laquelle au moins l'un des supports pharmaceutiquement acceptables est un solide à la température ambiante et/ou présente un point de fusion dans la plage de 40 à 80°C et est choisi parmi le groupe constitué du polyéthylène-glycol 6000, d'huiles végétales, de graisses végétales, de triglycérides synthétiques partiels, de triglycérides synthétiques, de mélanges d'esters du glycérol et d'acides gras, de mélanges de mono-, de di- et de triglycérides, d'esters du polyoxyéthylène-glycérol et d'acide gras, d'acides gras, d'esters d'acide gras, de cire, de la paraffine et de leurs mélanges.

3. Composition selon la revendication 1, dans laquelle le support pharmaceutiquement acceptable est un polymère.

4. Composition selon la revendication 3, dans laquelle ledit polymère est un polymère hydrophile.

5. Composition selon la revendication 4, dans laquelle le polymère hydrophile est choisi parmi le groupe constitué d'une polyvinylpyrrolidone ; d'un polyacétate de vinyle ; d'un alcool polyvinylique ; d'un phtalate d'alcool polyvinylique ; d'un polyéthylène-glycol ; d'un polyoxyde d'éthylène ; de la gélatine ; d'un carbomère ; d'un copolymère de l'acide méthacrylique ; d'un copolymère d'ammoniométhacrylate ; de la cellulose, de la carboxyméthylcellulose ; de la méthylcellulose ; de l'hydroxyéthylcellulose ; de l'hydroxypropylméthylcellulose, de l'hydroxypropylcellulose ; de l'acétate-phtalate de cellulose ; du phtalate d'hydroxypropylméthylcellulose, de la crospovidone, du glycolate d'amidon sodique ; de la croscarmellose ; et de leurs copolymères, et de leurs mélanges.

6. Composition selon l'une quelconque des revendications 1 à 5, la composition comprenant en outre un oestrogène.

7. Composition selon l'une quelconque des revendications précédentes, ladite composition étant sous une forme galénique solide.

8. Procédé de préparation d'une composition telle que définie dans la revendication 1, comprenant les étapes consistant à
a) procurer de la Drospirénone et un ou plusieurs support(s) ; et
b) dissoudre complètement la Drospirénone dans l'un ou plusieurs support(s) ; et
c) sécher le mélange obtenu dans l'étape b).

9. Procédé selon la revendication 8, dans lequel l'étape de dissolution de la Drospiréone est réalisée par un moyen choisi parmi le groupe constitué d'un chauffage, d'un traitement par ultrasons, d'une mixtion vigoureuse, d'une agitation et d'une extrusion à l'état fondu.

10. Procédé selon l'une quelconque des revendications 8 ou 9, dans lequel au moins l'un des supports pharmaceutiquement acceptables est un solide à la température ambiante et/ou présente un point de fusion dans la plage de 40 à 80°C et est choisi parmi le groupe constitué du polyéthylène-glycol 6000, d'huile végétales, de graisses végétales, de triglycérides synthétiques partiels, de triglycérides synthétiques, de mélanges d'esters du glycérol et d'acides gras, de mélanges de mono-, de di- et de triglycérides, d'esters du polyoxyéthylène-glycérol et d'acide gras, d'acides gras, d'esters d'acide gras, de cire, de la paraffine et de leurs mélanges.

11. Procédé selon l'une quelconque des revendications 8 ou 9, dans lequel le support pharmaceutiquement acceptable est un polymère.

12. Procédé selon la revendication 11, dans lequel ledit polymère est un polymère hydrophile.

13. Procédé selon la revendication 12, dans lequel le polymère hydrophile est choisi parmi le groupe constitué d'une polyvinylpyrrolidone ; d'un polyacétate de vinyle ; d'un alcool polyvinylique ; d'un phtalate d'alcool polyvinylique ; d'un polyéthylène-glycol ; d'un polyoxyde d'éthylène ; de la gélatine ; d'un carbomère ; d'un copolymère de l'acide méthacrylique ; d'un copolymère d'ammonio-méthacrylate ; de la cellulose, de la carboxyméthylcellulose ; de la méthylcellulose ; de l'hydroxyéthylcellulose ; de l'hydroxypropylméthylcellulose, de l'hydroxypropylcellulose ; de l'acétate-phtalate de cellulose ; du phtalate d'hydroxypropylméthylcellulose, de la crospovidone, du glycolate d'amidon sodique ; de la croscarmellose ; et de leurs copolymères, et de leurs mélanges.
